(19) Europäisches Patentamt
European Patent Office
Office européen des brevets

(11) **EP 4 358 653 A1**

(12) **EUROPEAN PATENT APPLICATION**
published in accordance with Art. 153(4) EPC

(43) Date of publication:
**24.04.2024 Bulletin 2024/17**

(21) Application number: **22824476.0**

(22) Date of filing: **25.01.2022**

(51) International Patent Classification (IPC):
**H05H 7/04** (2006.01) **H05H 7/10** (2006.01)
**H05H 13/00** (2006.01) **A61N 5/10** (2006.01)

(52) Cooperative Patent Classification (CPC):
**A61N 5/10; H05H 7/04; H05H 7/08; H05H 7/10; H05H 13/00**

(86) International application number:
**PCT/JP2022/002575**

(87) International publication number:
**WO 2022/264475 (22.12.2022 Gazette 2022/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.06.2021 JP 2021098983**

(71) Applicant: **Hitachi, Ltd.**
**Tokyo 100-8280 (JP)**

(72) Inventors:
• **HORI, Chishin**
**Tokyo 100-8280 (JP)**
• **AOKI, Takamichi**
**Tokyo 100-8280 (JP)**
• **HAE, Takamitsu**
**Tokyo 100-8280 (JP)**

(74) Representative: **MERH-IP Matias Erny Reichl Hoffmann**
**Patentanwälte PartG mbB**
**Paul-Heyse-Strasse 29**
**80336 München (DE)**

(54) **PARTICLE BEAM ACCELERATOR AND PARTICLE BEAM THERAPY SYSTEM**

(57)     A dynamic magnetic field feed device 52 feeds a magnetic field at predetermined timing to a predetermined region through which an ion beam having desired energy circulating in an acceleration space passes, and displaces a circular orbit of the ion beam having the desired energy. An extraction channel 1019 is arranged on an outer periphery of a magnetic pole 9. A position O1 where an ion introduction device introduces ions into the acceleration space is a position closer to the extraction channel 1019 relative to a center O2 of the magnetic pole. A region to which the dynamic magnetic field feed device 52 feeds a magnetic field is a region closer to an opening 1019a of the extraction channel 1019 relative to the position O1 where ions are introduced, and the magnetic field to be fed is a magnetic field in a direction where the main magnetic field is strengthened. Due to this, it is possible to provide a variable beam energy accelerator capable of improving time responsiveness of a beam related to extraction control and performing more accurate extraction control.

FIG. 4

**Description**

Technical Field

The present invention relates to a particle beam accelerator and a particle therapy system including the particle beam accelerator.

Background Art

Particle therapy is a type of radiation therapy, and is a treatment method in which a tumor is irradiated with an ion beam (hereinafter, abbreviated as a beam for simplicity) such as a proton beam or a carbon beam to destroy cancer cells. A particle therapy system for performing particle therapy includes an ion source that generates ions, a particle beam accelerator (hereinafter, abbreviated as an accelerator for simplicity) that accelerates a large amount of ions, a beam transport system that transports a beam to a treatment room, a rotating gantry that changes a beam irradiation direction with respect to a tumor, an irradiation system that irradiates a tumor with a beam from the rotating gantry, and a control system that controls these components.

PTL 1 discloses an accelerator having a structure in which a main magnetic field is formed between a pair of magnets, ions are injected from an ion source onto between the magnets, the ions are accelerated by an acceleration electrode, a radius of a circular orbit is gradually increased, and the ions accelerated to have predetermined energy are extracted along a beam extraction path to the outside. In the accelerator of PTL 1, the main magnetic field is formed so that the centers of a plurality of annular circular orbits are gradually shifted as being accelerated, and an aggregation region in which the circular orbits are close to one another and a sparse region in which the circular orbits are separated from one another are formed. A magnetic field (kicker magnetic field) is selectively fed only to a specific position in the radial direction of the magnet from the massless septum coil arranged so as to cover the sparse region of the circular orbit, the beam of a specific circular orbit is shifted from the design orbit and made injected on a septum magnet arranged in the vicinity of the aggregation region on a halfway downstream. The septum magnet further feeds a magnetic field to the injected beam to bend the beam, guides the beam to a beam extraction path, and extracts the beam from the accelerator.

Citation List

Patent Literature

PTL 1: JP 6714146

Summary of Invention

Technical Problem

PTL 1 discloses a method for extracting a variable energy beam, the method including: (1) generating a region where beam orbits are aggregated by an eccentric main magnetic field distribution; (2) energizing a massless septum coil with respect to a beam of energy desired to extract and feeding a dynamic magnetic field (magnetic field that is on/off in a time axis direction) to displace the beam orbit; and (3) causing a beam having reached a septum magnet arranged in the vicinity of the aggregation region to leave from a circular orbit by a magnetic field fed by the septum magnet. In this beam extraction process, if the excitation time of the massless septum in the process (2) can be shortened, the time responsiveness of the beam extraction control is improved.

An object of the present invention is to provide a variable beam energy accelerator capable of improving time responsiveness of a beam related to extraction control and performing more accurate extraction control.

Solution to Problem

In order to achieve the above object, a particle beam accelerator of the present invention includes a main magnetic field generation device, an ion introduction device, a radiofrequency acceleration system, a dynamic magnetic field feed device, a regenerator gradient magnetic field region, and an extraction channel. The main magnetic field generation device includes a pair of magnetic poles each having a circular outer periphery, and generates a main magnetic field in an acceleration space between the pair of magnetic poles. The ion introduction device introduces ions into the acceleration space. The radiofrequency acceleration system feeds a radiofrequency electric field to ions to accelerate the ions, forms an ion beam circulating in the acceleration space, and accelerates the ion beam to have desired energy. The dynamic magnetic field feed device feeds a magnetic field at predetermined timing to a predetermined region through which the ion beam having the desired energy circulating in the acceleration space passes, and displaces the circular orbit of the ion beam having the desired energy. The regenerator gradient magnetic field region is formed at a predetermined position in a peripheral edge portion of each of the magnetic poles. The extraction channel is arranged on an outer periphery of the magnetic pole, includes an opening for capturing an ion beam having the desired energy, and guides the captured ion beam from the acceleration space to the outside. The position where the ion introduction device introduces ions into the acceleration space is a position close to the extraction channel relative to the center of the magnetic pole. The region to which the dynamic magnetic field feed device feeds the magnetic field is a region covering a position along the circumferential direction of the circular orbit of the ion beam having the desired energy, the position in a direction where the circular orbit is displaced. In the regenerator gradient magnetic field region, a gradient magnetic field that increases toward the outer periphery of the magnetic pole is formed. The position provided with the regenerator gradient magnetic field region is a position where the ion beam having the desired energy in the circular orbit does not pass before the magnetic field of the dynamic magnetic field feed device is fed, and the ion beam in the circular orbit displaced by feeding the magnetic field of the dynamic magnetic field feed device passes.

Advantageous Effects of Invention

According to the present invention, since the dynamic magnetic field fed for the variable energy beam extraction can be reduced, the time responsiveness of the beam extraction control is improved, and the extraction control can be performed with higher accuracy.

Brief Description of Drawings

[FIG. 1] FIG. 1 is a block diagram illustrating an overall configuration of a particle therapy system in First embodiment of the present invention.
[FIG. 2] FIG. 2 is a perspective view of a main magnetic field magnet 1 arranged in an accelerator of the particle therapy system in First embodiment.
[FIG. 3] FIG. 3 is a cross-sectional view of a vertical plane position of the main magnetic field magnet 1 in First embodiment.
[FIG. 4] FIG. 4 is a cross-sectional view of a median plane position of the main magnetic field magnet 1 in First embodiment.
[FIG. 5] FIG. 5 is a graph showing a distribution of a main magnetic field of the main magnetic field magnet 1 in First embodiment.
[FIG. 6] FIG. 6 is a view illustrating circular orbits of the main magnetic field magnet 1 in an acceleration space in First embodiment.
[FIG. 7] FIG. 7 is a graph showing a distribution of magnetic fields formed by bump coils 52 and 53 of the main magnetic field magnet 1 in First embodiment.
[FIG. 8] FIG. 8 is a cross-sectional view of a median plane position of a main magnetic field magnet in Second embodiment.

Description of Embodiments

Hereinafter, embodiments of the present invention will be described with reference to the drawings.

<<<First embodiment>>>

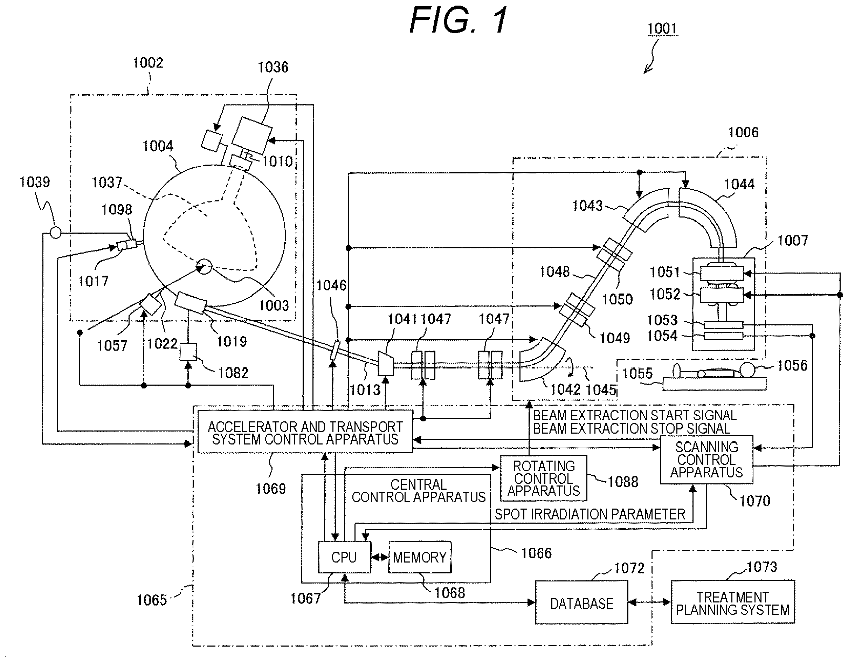

An overall configuration of the particle therapy system of First embodiment will be described with reference to FIG. 1. In the present embodiment, accelerating ions are hydrogen ions, that is, protons, and the beam energy extracted for use in treatment is approximately 70 MeV to 225 MeV.
In FIG. 1, a particle therapy system 1001 is installed on a floor surface of a building (not illustrated). The particle therapy system 1001 includes an ion beam generator 1002, a beam transport system 1013, a rotating gantry 1006, an irradiation apparatus 1007, and a control system 1065.

<Ion Beam Generator 1002>

The ion beam generator 1002 includes an ion source 1003 and an accelerator 1004 to which the ion source 1003 is connected. Details of the accelerator 1004 will be described later.

<Beam Transport System 1013>

The beam transport system 1013 has a beam path 1048 reaching the irradiation apparatus 1007, and is configured by arranging a plurality of quadrupole magnets 1046, a bending magnet 1041, a plurality of quadrupole magnets 1047, a bending magnet 1042, quadrupole magnets 1049 and 1050, and bending magnets 1043 and 1044 in this order from the accelerator 1004 toward the irradiation apparatus 1007 in this beam path 1048.

A part of the beam path 1048 of the beam transport system 1013 is installed in the rotating gantry 1006, and the bending magnet 1042, the quadrupole magnets 1049 and 1050, and the bending magnets 1043 and 1044 are also installed in the rotating gantry 1006. The beam path 1048 is connected to an extraction channel 1019 provided in the accelerator 1004.

<Rotating Gantry 1006>

The rotating gantry 1006 is configured to be rotatable about a rotation axis 1045, and is a rotation device that swivels the irradiation apparatus 1007 about the rotation axis 1045.

The irradiation apparatus 1007 includes two scanning magnets 1051 and 1052, a beam position monitor 1053, and a dose monitor 1054. These scanning magnets 1051 and 1052, the beam position monitor 1053, and the dose monitor 1054 are arranged along a center axis of the irradiation apparatus 1007, that is, a beam axis. The scanning magnets 1051 and 1052, the beam position monitor 1053, and the dose monitor 1054 are arranged in a casing (not illustrated) of the irradiation apparatus 1007.

The beam position monitor 1053 and the dose monitor 1054 are arranged downstream of the scanning magnets 1051 and 1052. The scanning magnet 1051 and the scanning magnet 1052 each bend the ion beam and scan the ion beam in directions orthogonal to each other in a plane vertical to the center axis of the irradiation apparatus 1007. The beam position monitor 1053 measures the passing position of the beam to be irradiated. The dose monitor 1054 measures the dose of the beam to be irradiated.

The irradiation apparatus 1007 is attached to the rotating gantry 1006 and is arranged downstream of the bending magnet 1044.

<Treatment Table 1055>

On the downstream side of the irradiation apparatus 1007, a treatment table 1055 on which a patient 1056 lies is arranged so as to face the irradiation apparatus 1007.

<Control System 1065>

The control system 1065 includes a central control apparatus 1066, an accelerator and transport system control apparatus 1069, a scanning control apparatus 1070, a rotation control apparatus 1088, and a database 1072.

The central control apparatus 1066 includes a central processing unit (CPU) 1067 and a memory 1068 connected to the CPU 1067. The accelerator and transport system control apparatus 1069, the scanning control apparatus 1070, the rotation control apparatus 1088, and the database 1072 are connected to the CPU 1067 in the central control apparatus 1066.

The particle therapy system 1001 further includes a treatment planning system 1073. The treatment planning system 1073 is connected to the database 1072. The treatment planning system 1073 generates treatment planning that defines the irradiation energy, the irradiation angle, and the like of the particle beam, and stores the treatment planning in the database 1072.

Based on this treatment planning, the central control apparatus 1066 controls the accelerator and transport system control apparatus 1069, the scanning control apparatus 1070, and the rotation control apparatus 1088, and irradiation is executed.

Specifically, the CPU 1067 of the central control apparatus 1066 reads various operation control programs related to irradiation of each device constituting the particle therapy system 1001 from the treatment planning stored in the database 1072, executes the read programs, and outputs a command via the accelerator and transport system control apparatus 1069, the scanning control apparatus 1070, and the rotation control apparatus 1088, thereby controlling the operation of each device in the particle therapy system 1001.

Note that the control processing of the operation to be executed may be integrated into one program, may be divided into a plurality of programs, or may be a combination thereof. Some or all programs may be achieved by dedicated hardware or may be modularized. Furthermore, the various programs may be installed in each computer by a program distribution server or an external storage medium.

Each control apparatus may be an independent device connected by a wired or wireless network, or two or more control apparatuses may be integrated.

<Details of Accelerator 1004>

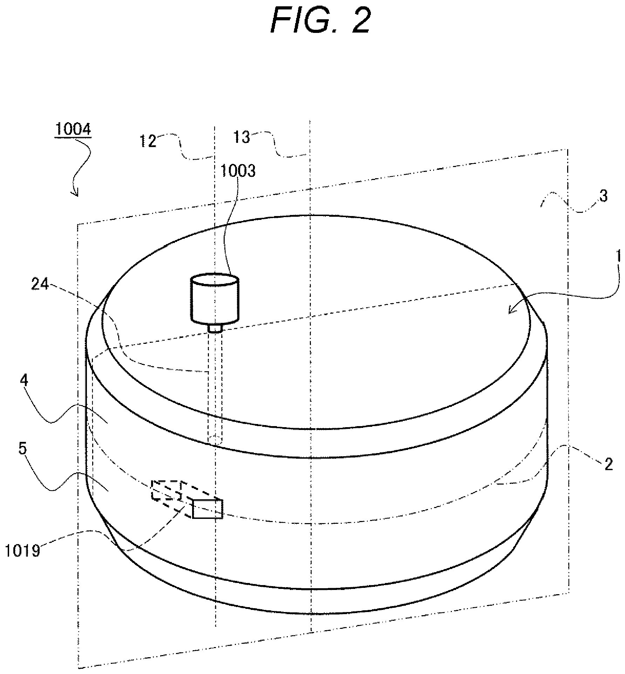

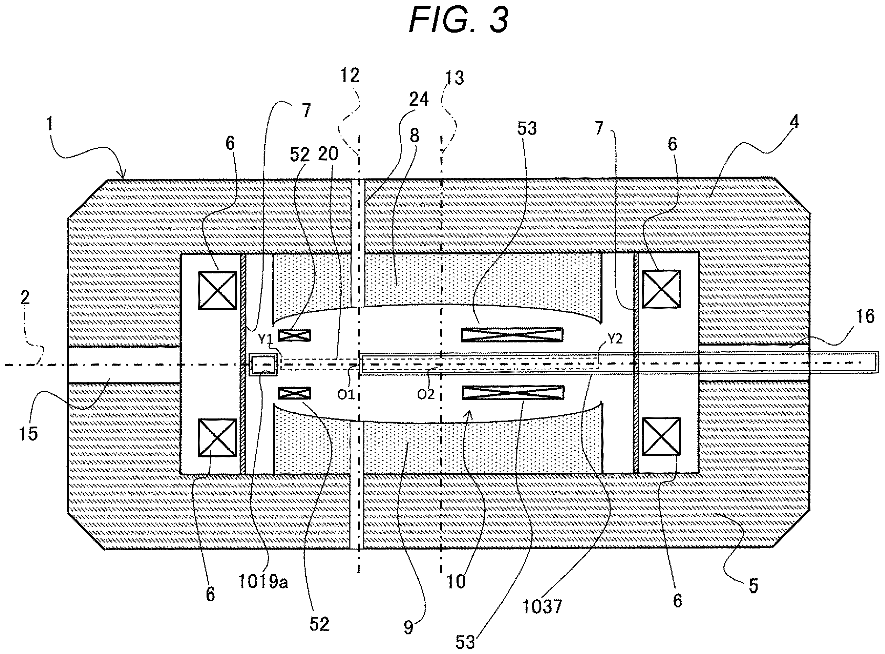

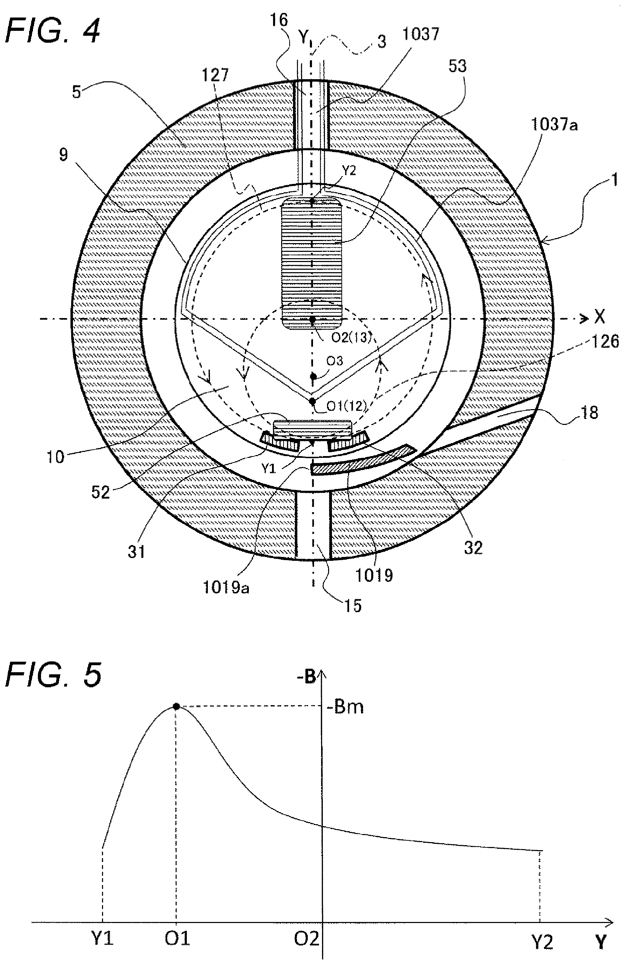

Next, the circular accelerator 1004 of the ion beam generator 1002 will be described in detail with reference to FIGS. 1 to 4. FIG. 2 is a perspective view of the main magnetic field magnet 1 of the circular accelerator 1004, FIG. 3 is a cross-sectional view of the main magnetic field magnet 1 at a position of a vertical plane 3, and FIG. 4 is a cross-sectional view of the main magnetic field magnet 1 at a position of a median plane 2.

The circular accelerator 1004 includes the main magnetic field magnet (main magnetic field generation device) 1 illustrated in FIGS. 2 to 4, a radiofrequency acceleration cavity 1037 arranged in the main magnetic field magnet 1, and the extraction channel 1019. The ion source 1003 is arranged on the main magnetic field magnet 1. As illustrated in FIG. 1, a radiofrequency power supply 1036, a beam current measurement device 1098, and a coil excitation power supply 1057 are connected to the circular accelerator 1004.

(Main Magnetic Field Magnet 1)

Details of the main magnetic field magnet 1 constituting the accelerator 1004 will be described.

As illustrated in FIG. 2, the main magnetic field magnet 1 is configured to include an upper return yoke 4 and a lower return yoke 5 having a substantially disk shape when viewed from the vertical direction, a pair of upper magnetic pole 8 and lower magnetic pole 9, and a coil 6.

The upper return yoke 4 and the lower return yoke 5 have substantially vertically symmetrical shapes with respect to the median plane 2. This median plane 2 generally passes through the vertical direction center of the main magnetic field magnet 1 and substantially coincides with an orbital plane drawn by the beam during acceleration.

The upper return yoke 4 and the lower return yoke 5 have substantially plane-symmetrical shapes with respect to the vertical plane 3, which is a plane vertical to the median plane 2 and passing through the center of the main magnetic field magnet 1 with respect to the median plane 2. In FIG. 2, an intersection of the median plane 2 with respect to the main magnetic field magnet 1 is indicated by an alternate long and short dash line, and an intersection of the vertical plane 3 with respect to the main magnetic field magnet 1 is indicated by a broken line.

As illustrated in FIG. 3, in a space surrounded by the upper return yoke 4 and the lower return yoke 5, the coil 6 is arranged plane-symmetrically with respect to the median plane 2. The coil 6, which is a superconducting coil, is installed inside a cryostat (not illustrated), and is cooled by a refrigerant such as liquid helium or heat transfer from a refrigerator (not illustrated). The coil 6 is connected to the coil excitation power supply 1057 by a coil draw out wire 1022 illustrated in FIG. 1.

A vacuum container 7 is provided inside the coil 6 in the space surrounded by the upper return yoke 4 and the lower return yoke 5. Inside the vacuum container 7, the upper magnetic pole 8 and the lower magnetic pole 9 are arranged plane-symmetrically across the median plane 2, and are coupled to the upper return yoke 4 and the lower return yoke 5, respectively.

The upper return yoke 4 and the lower return yoke 5, and the upper magnetic pole 8 and the lower magnetic pole 9 are made of, for example, pure iron with a reduced impurity concentration, low carbon steel, or the like. The vacuum container 7 is made of stainless steel or the like. The coil 6 is made of a superconducting wire rod using a superconductor such as niobium titanium.

The main magnetic field magnet 1 having the above-described configuration forms a main magnetic field that feeds a vertical magnetic field with respect to an acceleration space 20 about the median plane 2. The main magnetic field is substantially uniform in the median plane 2, but a slight intensity distribution is formed. That is, as in FIG. 5, the intensity of the main magnetic field is the largest at a predetermined position O1 shifted in the Y axis direction from a center 02 of the upper magnetic pole 8 and the lower magnetic pole 9, and the magnetic field intensity gradually decreases toward the outer peripheries of the upper magnetic pole 8 and the lower magnetic pole 9.

The extraction channel 1019 is arranged on the outer periphery of the magnetic poles 8 and 9 on the Y axis close to a center O1 of the main magnetic field distribution, that is, outside the acceleration space. The extraction channel 1019 has an opening 1019a in the vicinity of the Y axis, and captures and guides, from the acceleration space 20 to the outside, an ion beam having desired energy from this opening 1019a. The ion beam having the desired energy is shifted in the orbital position by the bump coil (dynamic magnetic field generator) 52 described later from the state of circulating in the acceleration space 20, deviates from the orbit by a gradient magnetic field magnet 31 (peeler), and enters the extraction channel 1019. This will be described in detail later.

The extraction channel 1019 includes a magnet, feeds a magnetic field to the ion beam captured from the opening 1019a to adjust the ion beam, and sends the ion beam to the beam transport system 1013. The upper return yoke 4 and the lower return yoke 5 are provided with a through hole 18, and a front end of the beam transport system 1013 is installed.

A power supply wire to the magnet is drawn out from a through hole 15 provided in the upper return yoke 4 and the lower return yoke 5, and is connected to an extraction channel power supply 1082 illustrated in FIG. 1.

(Ion Source (Ion Introduction Device) 1003)

On the main magnetic field magnet 1, the ion source 1003 is installed here as an ion introduction device. The upper return yoke 4 and the upper magnetic pole 8 are provided with a through hole 24 guiding ions of the ion source 1003 to the position O1 of the acceleration space 20. A center axis (ion injection axis) 12 of the through hole 24 is vertical to the median plane 2 and passes through the position O1.

The ion source 1003 is arranged above the through hole 24, and introduces ions to the position O1 where the main magnetic field intensity is maximum through the through hole 24. Since the main magnetic field has a maximum value at the position O1 where the ion beam is injected and monotonously decreases therefrom toward the outer periphery, the beam circulating motion can be stabilized by the principle of weak focusing.

Note that the ion source 1003 may be installed inside the main magnetic field magnet 1. In this case, the through hole 24 is unnecessary.

(Radiofrequency Acceleration Cavity 1037)

The radiofrequency acceleration cavity 1037 includes a pair of dee electrodes 1037a arranged across the median plane 2. The dee electrode 1037a has a fan shape. A vertex (center) of the fan shape is positioned in the vicinity of the position O1 where the main magnetic field intensity is maximum, and is arranged so as to cover a half circumference of the beam orbit including a position O2 of the centers of the upper magnetic pole 8 and the lower magnetic pole 9.

A ground electrode (not illustrated) is arranged so as to face a radial end surface of the fan-shaped dee electrode 1037a, and an acceleration electric field that accelerates the ion beam is formed between the radial end surface of the dee electrode 1037a and the ground electrode.

By forming the dee electrode 1037a into a fan shape having the position O1 as a vertex, it is possible to feed the acceleration electric field to a position where the circulating direction of the circulating ion beam becomes parallel to the electric field, that is, an axis parallel to the X axis passing through the center of each circular orbit intersects each circular orbit.

The radiofrequency acceleration cavity 1037 is drawn out through a through hole 16 provided along the Y axis direction between the upper return yoke 4 and the lower return yoke 5, and is connected to a waveguide 1010. The radiofrequency power supply 1036 is connected to the waveguide 1010.

The radiofrequency power supply 1036 inputs power to the radiofrequency acceleration cavity 1037 through the waveguide 1010. This excites a radiofrequency electric field that accelerates the beam between the dee electrode 1037a and the ground electrode.

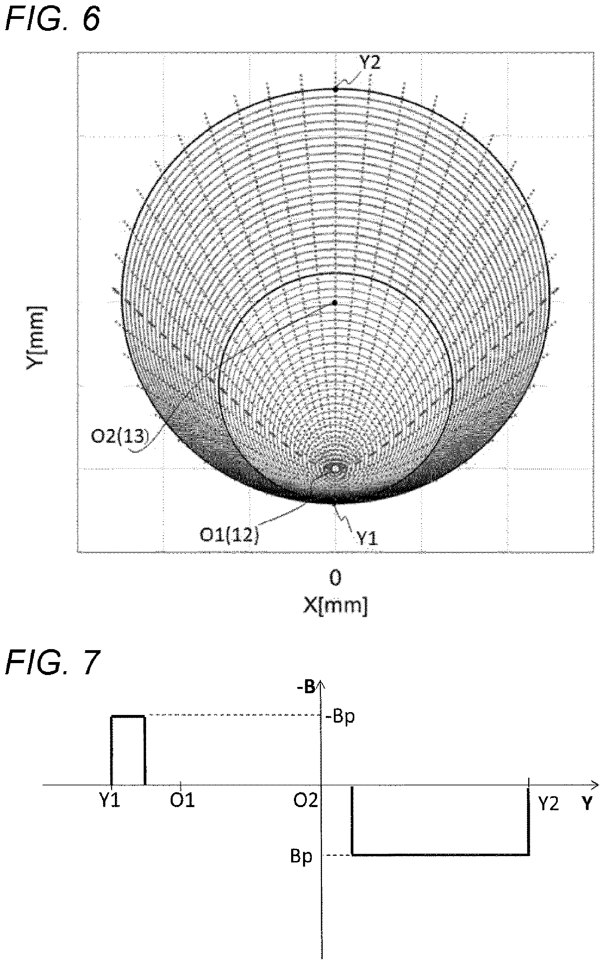

In the present embodiment, as in FIG. 6, the orbit radius of the circular orbit gradually increases with the acceleration of the beam. Therefore, the radiofrequency acceleration cavity 1037 modulates the resonance frequency in accordance with the energy of the beam. The modulation of the frequency is performed by adjusting the inductance or the capacitance of the radiofrequency acceleration cavity 1037. As an adjustment method of the inductance and the capacitance of the radiofrequency acceleration cavity 1037, a known method can be used. For example, in the case of adjusting the capacitance, a variable capacitance capacitor is connected to the radiofrequency cavity to control the capacitance.

(Sparseness and Denseness of Circular Orbits)

With the above configuration, the ions introduced from the ion source 1003 are excited by the radiofrequency electric field to become an ion beam, and circulate in the acceleration space 20. At this time, as illustrated in FIG. 5, the main magnetic field in the acceleration space 20 has a distribution in which the main magnetic field is maximum at the position O1 shifted from the position O2 of a center axis 13 of the magnetic poles 8 and 9 and gradually decreases as approaching the outer peripheries of the magnetic poles 8 and 9, and therefore the beam having the lowest energy circulates along the orbit about the position O1. As the beam is accelerated by the radiofrequency electric field, the orbit radius increases, and the center of the orbit gradually approaches the position O2 of the center axis 13 of the magnetic poles 8 and 9. A circular orbit 127 of the beam of the maximum energy has a shape substantially along the outer circumferences of the magnetic poles 8 and 9, and its center O2 generally coincides with the center axis 13 of the magnetic poles 8 and 9.

Due to this, as illustrated in FIG. 6, the circular orbits become dense between the position O1 and a position Y1 of the end portion of the acceleration space 20 in the Y axis direction, and the circular orbits become sparse between the position O1 and a position Y2 of the end portion in the Y axis direction on the opposite side across the position O2 of the centers of the magnetic poles 8 and 9.

In the present embodiment, it is possible to extract a beam having energy in a predetermined range using such sparseness and denseness of the orbits. For example, as in FIG. 4, the center of the circular orbit 127 of the beam corresponding to the highest energy among the extracted beams substantially coincides with the position O2 of the center axis 13 of the magnetic poles 8 and 9. In the circular orbit 126 of the beam corresponding to the lowest energy among the extracted beams, a center O3 is on a line segment connecting the position O2 and the position O1 of the maximum intensity of the main magnetic field distribution.

(Dynamic Magnetic Field Feed Devices (Bump Coils) 52 and 53)

In the present embodiment, the pair of bump coils 52 as the first dynamic magnetic field feed device and the pair of bump coils 53 as the second dynamic magnetic field feed device are each arranged outside the radiofrequency acceleration cavity 1037 at plane-symmetrical positions across the median plane 2. The bump coil 52 and the bump coil 53 feed a magnetic field at predetermined timing to a predetermined region through which the ion beam having desired energy circulating in the acceleration space passes, and displace the circular orbit of the ion beam having desired energy in the Y axis direction so as to approach the position Y1.

The bump coil 52 is arranged so as to feed a magnetic field to a region of the acceleration space 20 closer to the extraction channel 109 relative to the position O1 where the ion source 1003 introduces ions into the acceleration space 20. As illustrated in FIG. 7, the orientation of the magnetic field fed by the bump coil 52 is a direction where the main magnetic field is strengthened.

Specifically, the bump coil 52 generates a magnetic field in a direction where the main magnetic field is strengthened at the position Y1 where the opening 1019a of the extraction channel 1019 is arranged and in the vicinity of the position Y1. It is sufficient that a magnetic field can be fed to a position where the ion beam having energy desired to extract comes closest to the position Y1 and the vicinity of the position. Therefore, the bump coil 52 is configured so as to feed a magnetic field from the circular orbit 126 of the ion beam having the minimum energy desired to extract to the position where the circular orbit 127 of the ion beam having the maximum energy comes closest to the position Y1 and the vicinity of the position.

On the other hand, the bump coil 53 is arranged at a position where a magnetic field is fed to the acceleration space 20 on the opposite side of the bump coil 52 across the position O1 where the ion source 1003 introduces ions into the acceleration space 20. As illustrated in FIG. 7, the orientation of the magnetic field fed by the bump coil 53 is a direction where the main magnetic field is weakened (orientation opposite to the main magnetic field).

Specifically, the bump coil 53 generates a magnetic field in a direction where the main magnetic field is weakened at the position Y2 where the phase of betatron oscillation is shifted by $\pi$ with respect to the position Y1 and the vicinity of the position Y2. It is sufficient that a magnetic field can be fed to a position where the ion beam having energy desired to extract comes closest to the position Y2 and the vicinity of the position. Therefore, the bump coil 53 is configured such that the range in which the bump coil 53 feeds the magnetic field is a position where the circular orbit from the circular orbit 126 of the ion beam having the minimum energy desired to extract to the circular orbit 127 of the ion beam having the maximum energy comes closest to the position Y2 and the vicinity of the position. Since the vicinity of the position Y2 is a region where the circular orbits of the ion beam become sparse, the area of the region where the bump coil 53 feeds the magnetic field becomes larger than that of the bump coil 52.

The positions where the bump coils 52 and 53 should be arranged and the like will be described later using a mathematical formula.

In the device of the present embodiment, the bump coil 52 and the bump coil 53 are arranged, but only the bump coil 52 alone may be arranged. The bump coil 52 alone can bring the circular orbit having desired energy close to the position Y1.

(Gradient Magnetic Field Magnet 31)

The gradient magnetic field magnet (peeler) 31 and a gradient magnetic field magnet (regenerator) 32 are arranged at predetermined positions of the peripheral edge portions of the magnetic poles 8 and 9 to form a peeler gradient magnetic field region and a regenerator gradient magnetic field region, respectively. The extraction channel 1019 is positioned circumferentially between the gradient magnetic field magnet 31 and the gradient magnetic field magnet 32 and radially outside the gradient magnetic field magnet 31 and the gradient magnetic field magnet 32.

The peeler gradient magnetic field region formed by the gradient magnetic field magnet 31 is set to a region through which the ion beam in the circular orbit displaced by the magnetic field of the bump coils 52 and 53 passes. The magnetic field distribution of the peeler gradient magnetic field region is a gradient magnetic field in which the magnetic field decreases radially outward.

The regenerator gradient magnetic field region formed by the gradient magnetic field magnet 32 is set to a region through which the ion beam in the circular orbit displaced by the magnetic field of the bump coils 52 and 53 passes that is a region through which the ion beam that has not injected the extraction channel passes. The regenerator gradient magnetic field region is a gradient magnetic field in which the magnetic field increases radially outward.

Note that the gradient magnetic field magnets 31 and 32 may be formed integrally with the lower magnetic pole 9, or may be engaged with the lower magnetic pole 9 by a known method such as welding or bolting after being manufactured as separate members.

It is also possible to form the peeler gradient magnetic field region and the regenerator gradient magnetic field region by further adding a magnetic body to the surface of the lower magnetic pole 9 or by processing the facing surface shape of the lower magnetic pole 9 without arranging the gradient magnetic field magnets 31 and 32.

Note that the peeler gradient magnetic field region, which is a gradient magnetic field that decreases radially outward, can be omitted. Since the magnetic field in the region to the yoke inner circumferential surface outside the magnetic pole outer circumferential surface decreases radially outward, the magnetic field can also be used as a peeler gradient magnetic field region.

(Beam Current Measurement Device 1098)

The accelerator 1004 includes the beam current measurement device 1098 that measures the current of the accelerated internal beam. The beam current measurement device 1098 includes a movement device 1017 and a position detector 1039.

<<Operation of Accelerator 1004>>

The operation of each unit when ions are accelerated by the accelerator 1004 and an ion beam having desired energy is extracted will be described.

Under the control of the central control apparatus 1066, the accelerator and transport system control apparatus 1069 causes the ion source 1003 to generate ions and introduces the ions into the position O1 of the median plane 2 through the through hole 24. The accelerator and transport system control apparatus 1069 generates an acceleration electric field by the radiofrequency acceleration cavity 1037, accelerates ions, forms an ion beam, and further accelerates. The ion beam increases in energy while performing circulating motion.

At the timing when the ion beam reaches the energy desired to extract, the accelerator and transport system control apparatus 1069 turns off the radiofrequency acceleration cavity 1037 and turns on the bump coil 52 and the bump coil 53. Due to this, the circulating ion beam is superimposed on the main magnetic field, and the dynamic magnetic field is fed.

Due to this, the beam orbit of the ion beam is displaced in the radial direction (direction approaching the position Y1), and the beam passes through the region where the gradient magnetic field formed by the gradient magnetic field magnet 31 and the gradient magnetic field magnet 32 acts.

Then, resonance of horizontal betatron oscillation called 2/2 resonance is generated, and the ion beam diverges in the horizontal direction and reaches the opening 1019a of the extraction channel 1019. By the extraction channel 1019, the ion beam is completely separated from the circular orbit and extracted to the outside of the accelerator 1004 through the through hole 18.

In the above beam extraction process, the magnitude of the beam orbit displacement generated by the bump coil 52 and the bump coil 53 is sufficient to be a distance from the equilibrium orbit to such an extent that the beam passes through the region where the gradient magnetic field magnet 31 and the gradient magnetic field magnet 32 act, and it is not necessary to generate a displacement large enough to reach the extraction channel 1019 installed radially outside the magnetic pole.

Therefore, the magnitude of the dynamic magnetic field fed by the bump coil 52 and the bump coil 53 can be reduced by about 10 times as compared with the massless septum of PTL 1.

Furthermore, since the bump coils are installed in two places such as the bump coil 52 is installed in the aggregation region of the beam orbit and the bump coil 53 is installed in the enlarged region of the beam orbit, it is possible to cause the beam displacement twice as large as the case where the bump coil is installed in one place.

As described above, in the present embodiment, since the excitation amount of the bump coils 52 and 53 can be reduced, the time response from the ON of the bump coil 52 to the beam extraction is improved. That is, beam extraction control with higher accuracy can be performed.

**<<Magnitude and Range of Magnetic Field of Bump Coils 52 and 53>>**

The relationship between the magnitude of the dynamic magnetic field fed by the bump coil 52 and the bump coil 53 and the displacement amount of the orbit of the ion beam will be further described using a mathematical formula.

When the circumferential position along the equilibrium orbit of the ion beam is expressed by s, a beam displacement amount $\delta$ at the position s when a unit amount of the magnetic field is added to s = s0 is expressed by Formula (1) in the range of linear optics.

[mathematical formula 1]

$$\delta = \frac{\sqrt{\beta(s)\beta\left(s_0\right)}}{2\sin\pi\nu}\cos\left(\pi\nu - \left|\psi(s) - \psi\left(s_0\right)\right|\right) \qquad \cdots (1)$$

Where $\beta$ is the horizontal betatron amplitude of the beam, and $\nu$ is the horizontal betatron oscillation frequency. s and s0 are circumferential positions. $\psi(s)$ - $\psi(s0)$ indicates a phase difference of the betatron oscillation.

In the present embodiment, a gradient magnetic field by the gradient magnetic field magnets 31 and 32 is caused to act on the beam, and the beam is extracted using a horizontal resonance of the beam called 2/2 resonance. Therefore, the horizontal betatron oscillation frequency $\nu$ is designed to be close to 1. This means that the phase of the betatron oscillation increases by approximately $2\pi$ during one round of the beam.

Therefore, when s and s0 are shifted substantially by half, the phase difference $\psi(s)$ - $\psi(s0)$ becomes substantially $\pi$, and the argument of cos becomes substantially 0.

When s and s0 match, the argument of cos becomes substantially $\pi$.

Therefore, when a position where the beam displacement is desired to maximize is s, it is efficient that the circumferential position s0 of the magnetic field to be added is the same as s, or a position where the phase is shifted by $\pi$ from s, and the vicinity of the position.

That is, when it is desired to generate beam displacement radially outward at the position s (the position Y1 of the embodiment), a magnetic field in a direction where the main magnetic field is strengthened may be added to the position s (the position Y1) and the vicinity thereof, and a magnetic field in a direction where the main magnetic field is weakened may be added to the position (the position Y2) where the phase is shifted by $\pi$ from the position s (the position Y1) and the vicinity thereof.

In order to achieve this, in the present embodiment, the bump coil 52 that generates a magnetic field in a direction where the main magnetic field is strengthened is arranged at the position Y1 where the opening 1019a of the extraction channel 1019 is arranged and in the vicinity thereof. Specifically, the bump coil 52 only needs to be able to feed a magnetic field to the ion beam having energy desired to extract. Therefore, the bump coil 52 is configured so as to feed a magnetic field from the circular orbit 126 of the ion beam having the minimum energy desired to extract to the position where the circular orbit 127 of the ion beam having the maximum energy comes closest to the position Y1 and the vicinity of the position.

The bump coil 53 is arranged so as to cover the position Y2 where the phase of the betatron oscillation is shifted by $\pi$ with respect to the position Y1 and the vicinity thereof, and generates a magnetic field in a direction where the main magnetic field is weakened. Specifically, the bump coil 53 is configured such that the range in which the bump coil 53 feeds the magnetic field is a position where the circular orbit 127 of the ion beam having the maximum energy comes closest to the position Y2 from the circular orbit 126 of the ion beam having the minimum energy desired to extract and the vicinity of the position. Since the vicinity of the position Y2 is a region where the circular orbits of the ion beam become sparse, the area of the region where the bump coil 53 feeds the magnetic field becomes larger than that of the bump coil 52.

As described above, according to the present embodiment, it is possible to provide a variable beam energy accelerator capable of improving time responsiveness of a beam related to extraction control and performing more accurate extraction control.

In the present embodiment, the beam displacement is generated by feeding the dynamic magnetic field, but, by processing the surface shapes of the upper magnetic pole 8 and the lower magnetic pole 9 at positions immediately above and immediately below the bump coil 52 and the bump coil 53 to locally narrow or widen the magnetic pole interval, it is possible to provide a configuration where a bump magnetic field is statically added to an extent that the beam is not destabilized during beam acceleration. In this case, it is possible to further reduce the dynamic magnetic field that should be fed by the bump coil 52 and the bump coil 53 for starting beam extraction.

<<<Second embodiment>>>

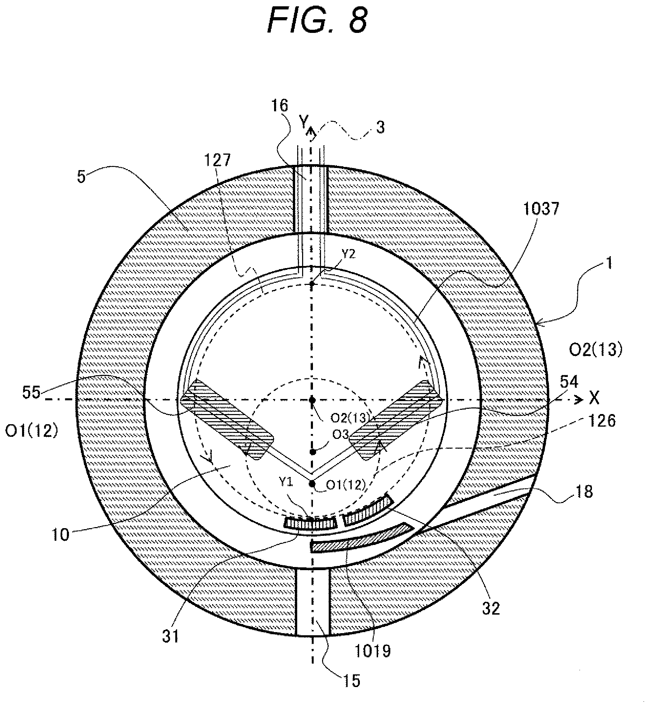

An accelerator of a particle therapy system in Second embodiment will be described with reference to FIG. 8.

FIG. 8 is a cross-sectional view of the accelerator of Second embodiment taken along the median plane 2.

While in First embodiment, the circular orbit of the ion beam having the desired energy is displaced in the Y axis direction by the bump coils 52 and 53 and brought close to the position Y1 to reach the peeler gradient magnetic field region formed by the gradient magnetic field magnet 31, in Second embodiment, the circular orbit is displaced in the X axis direction to reach the peeler gradient magnetic field region.

Specifically, in Second embodiment, the gradient magnetic field magnet 31 is arranged on the Y axis, and the gradient magnetic field magnet 32 is arranged on the right side (position shifted counterclockwise) thereof.

In order for the orbit of the ion beam desired to extract to pass through in a balanced manner the peeler gradient magnetic field region and the regenerator gradient magnetic field region on which the gradient magnetic field magnet 31 and the gradient magnetic field magnet 32 act, it is preferable to displace the orbit on the median plane 2 in a direction vertical to the vertical plane 3 (positive X direction).

Therefore, in Second embodiment, a bump coil 54 is arranged at a position shifted counterclockwise by 90 degrees from the position Y1 in each orbit from the circular orbit 126 of the ion beam having the minimum energy desired to extract to the circular orbit 127 of the ion beam having the maximum energy. Furthermore, a bump coil 55 is arranged at a position shifted counterclockwise by 180 degrees from the position of the bump coil 54. A magnetic field in a direction where the main magnetic field is strengthened is fed from the bump coil 54, and a magnetic field in a direction where the main magnetic field is weakened is fed from the bump coil 55.

Since the circular orbit of the ion beam is eccentric in the Y axis direction as illustrated in FIG. 6, the position shifted counterclockwise by 90 degrees from the position Y1 is shifted in the Y axis direction by each orbit, and therefore the bump coils 54 and 55 have shapes gradient with respect to the Y axis.

A position shifted by 90 degrees counterclockwise from the position Y1 and a position shifted by 180 degrees therefrom in each circular orbit of the ion beam coincides with a position where the radiofrequency acceleration cavity 1037 feeds the acceleration electric field, that is, a position of the radial end surface of the dee electrode 1037a. Therefore, the bump coil 54 and the bump coil 55 are arranged along the radial end surface of the dee electrode 1037a of the radiofrequency acceleration cavity 1037.

Due to this, by feeding a magnetic field in a direction where the main magnetic field is strengthened in the bump coil 54, and feeding a magnetic field in a direction where the main magnetic field is weakened in the bump coil 55, the beam orbit is displaced in the positive X direction.

The operation of the accelerator 1004 of Second embodiment will be described.

Similarly to First embodiment, the accelerator and transport system control apparatus 1069 causes the ion source 1003 to generate ions, causes the ions to be introduced into the position O1 of the median plane 2 through the through hole 24, and accelerates the ions by the radiofrequency acceleration cavity 1037.

At the timing when the ion beam reaches the energy desired to extract, the radiofrequency acceleration cavity 1037 is turned off, the bump coil 54 and the bump coil 55 are turned on, and the dynamic magnetic field is fed to the beam.

Due to this, the beam orbit is displaced in the +X direction, and the beam passes through the region where the gradient magnetic field magnet 31 and the gradient magnetic field magnet 32 act.

Then, resonance of horizontal betatron oscillation called 2/2 resonance is generated, and the beam diverges in the horizontal direction, reaches the extraction channel 1019, and reaches the opening 1019a of the extraction channel 1019. The beam is completely separated from the circular orbit and extracted to the outside of the accelerator 1004 through the through hole 18.

Note that, in Second embodiment, two of the bump coils 54 and 55 are arranged, but the orbit can be displaced in the X axis direction with only either one similarly to Second embodiment.

In the particle therapy system of Second embodiment, the configurations, operations, and effects other than those described above, and variations of modifications are similar to those of First embodiment, and thus the description thereof will be omitted.

Reference Signs List

1       main magnetic field magnet
2       median plane
3       vertical plane
4       upper return yoke
5       lower return yoke

| | |
|---|---|
| 6 | coil |
| 7 | vacuum container |
| 8 | upper magnetic pole |
| 9 | lower magnetic pole |
| 13 | center axis |
| 15 | through hole |
| 16 | through hole |
| 18 | through hole |
| 20 | acceleration space |
| 24 | through hole |
| 31 | gradient magnetic field magnet |
| 32 | gradient magnetic field magnet |
| 52 | bump coil |
| 53 | bump coil |
| 54 | bump coil |
| 55 | bump coil |
| 109 | extraction channel |
| 126 | circular orbit |
| 127 | circular orbit |
| 1001 | particle therapy system |
| 1002 | ion beam generator |
| 1003 | ion source |
| 1004 | accelerator |
| 1006 | rotating gantry |
| 1007 | irradiation apparatus |
| 1010 | waveguide |
| 1013 | beam transport system |
| 1017 | movement device |
| 1019 | extraction channel |
| 1019a | opening |
| 1022 | coil draw out wire |
| 1036 | radiofrequency power supply |
| 1037 | radiofrequency acceleration cavity |
| 1037a | dee electrode |
| 1039 | position detector |
| 1041 | bending magnet |
| 1042 | bending magnet |
| 1043 | bending magnet |
| 1044 | bending magnet |
| 1045 | rotation axis |
| 1046 | quadrupole magnet |
| 1047 | quadrupole magnet |
| 1048 | beam path |
| 1049 | quadrupole magnet |
| 1051 | scanning magnet |
| 1052 | scanning magnet |
| 1053 | beam position monitor |
| 1054 | dose monitor |
| 1055 | treatment table |
| 1056 | patient |
| 1057 | coil excitation power supply |
| 1065 | control system |
| 1066 | central control apparatus |
| 1068 | memory |
| 1069 | transport system control apparatus |
| 1070 | scanning control apparatus |
| 1072 | database |
| 1073 | treatment planning system |

| | |
|---|---|
| 1082 | extraction channel power supply |
| 1088 | rotation control apparatus |
| 1098 | beam current measurement device |
| O1 | position |
| O2 | position |
| O3 | position |
| Y1 | position |
| Y2 | position |

**Claims**

1. A particle beam accelerator, comprising:

a main magnetic field generation device including a pair of magnetic poles each having a circular outer periphery, the main magnetic field generation device generating a main magnetic field in an acceleration space between the pair of magnetic poles;
an ion introduction device that introduces ions into the acceleration space;
a radiofrequency acceleration system that feeds a radiofrequency electric field to the ions to accelerate the ions, forms an ion beam circulating in the acceleration space, and accelerates the ion beam to have desired energy;
a dynamic magnetic field feed device that feeds a magnetic field at predetermined timing to a predetermined region through which the ion beam having the desired energy circulating in the acceleration space passes, and displaces a circular orbit of the ion beam having the desired energy;
a regenerator gradient magnetic field region formed at a predetermined position in a peripheral edge portion of each of the magnetic poles; and
an extraction channel arranged on an outer periphery of the magnetic pole, the extraction channel including an opening through which the ion beam having the desired energy is captured, the extraction channel guiding, from the acceleration space to an outside, the ion beam having been captured,
wherein
a position where the ion introduction device introduces ions into the acceleration space is a position close to the extraction channel relative to a center of the magnetic pole,
a region to which the dynamic magnetic field feed device feeds a magnetic field is a region covering a position along a circumferential direction of the circular orbit of the ion beam having the desired energy, the position in a direction where the circular orbit is displaced,
a gradient magnetic field that increases toward the outer periphery of the magnetic pole is formed in the regenerator gradient magnetic field region, and
the position provided with the regenerator gradient magnetic field region is a position where the ion beam having the desired energy in the circular orbit does not pass before a magnetic field of the dynamic magnetic field feed device is fed, and an ion beam in the circular orbit displaced by feeding a magnetic field of the dynamic magnetic field feed device passes.

2. The particle beam accelerator according to claim 1, wherein

the circular orbit having the desired energy is any of a plurality of circular orbits in a predetermined energy range, and
a gradient of the gradient magnetic field formed in the regenerator gradient magnetic field region is larger than a gradient of the main magnetic field.

3. The particle beam accelerator according to claim 1, wherein a region to which the dynamic magnetic field feed device feeds a magnetic field is a region close to the opening of the extraction channel relative to a position where the ion introduction device introduces ions into the acceleration space, and the magnetic field to be fed is a magnetic field in a direction where the main magnetic field is strengthened.

4. The particle beam accelerator according to claim 1, wherein

a peeler gradient magnetic field region is formed at a predetermined position of the peripheral edge portion of the magnetic pole, and

the peeler gradient magnetic field region is a region through which the ion beam in the circular orbit displaced by the magnetic field of the dynamic magnetic field feed device passes, and a magnetic field decreases as approaching the outer periphery of the magnetic pole.

5. The particle beam accelerator according to claim 4, wherein the peeler gradient magnetic field region is formed of a magnetic body arranged in the peripheral edge portion of the magnetic pole.

6. The particle beam accelerator according to claim 4, wherein the peeler gradient magnetic field region is formed by processing the magnetic pole.

7. The particle beam accelerator according to claim 1, wherein the regenerator gradient magnetic field region is formed of a magnetic body arranged in the peripheral edge portion of the magnetic pole.

8. The particle beam accelerator according to claim 3, further comprising a second dynamic magnetic field feed device, wherein

the second dynamic magnetic field feed device is arranged at a position where a magnetic field is fed to a predetermined second region of the acceleration space on an opposite side across the position where the ion introduction device introduces ions into the acceleration space, with respect to the region where the dynamic magnetic field feed device feeds the magnetic field, and
a magnetic field fed by the second dynamic magnetic field feed device is a magnetic field in a direction where the main magnetic field is weakened.

9. The particle beam accelerator according to claim 1, wherein the direction where the circular orbit is displaced is a direction approaching the extraction channel.

10. The particle beam accelerator according to claim 1, wherein the direction where the circular orbit is displaced is a direction orthogonal to a line connecting the center of the magnetic pole and the opening of the extraction channel.

11. A particle beam accelerator, comprising:

a main magnetic field generation device including a pair of magnetic poles each having a circular outer periphery, the main magnetic field generation device generating a main magnetic field in an acceleration space between the pair of magnetic poles;
an ion introduction device that introduces ions into the acceleration space;
a radiofrequency acceleration system that feeds a radiofrequency electric field to the ions to accelerate the ions, forms an ion beam circulating in the acceleration space, and accelerates the ion beam to have desired energy;
a dynamic magnetic field feed device that feeds a magnetic field at predetermined timing to a predetermined region through which the ion beam having the desired energy circulating in the acceleration space passes, and displaces a circular orbit of the ion beam having the desired energy;
a regenerator gradient magnetic field region formed at a predetermined position in a peripheral edge portion of each of the magnetic poles; and
an extraction channel arranged on an outer periphery of the magnetic pole, the extraction channel including an opening through which the ion beam having the desired energy is captured, the extraction channel guiding, from the acceleration space to an outside, the ion beam having been captured,
wherein
a position where the ion introduction device introduces ions into the acceleration space is a position close to the extraction channel relative to a center of the magnetic pole,
the radiofrequency acceleration system includes a fan-shaped dee electrode about the position where ions are introduced into the acceleration space, and
the dynamic magnetic field feed device is arranged in a vicinity of a radial end surface of the fan-shaped dee electrode.

12. The particle beam accelerator according to claim 11, wherein

a gradient magnetic field that increases toward the outer periphery of the magnetic pole is formed in the regenerator gradient magnetic field region, and

the position provided with the regenerator gradient magnetic field region is a position where the ion beam having the desired energy in the circular orbit does not pass before a magnetic field of the dynamic magnetic field feed device is fed, and an ion beam in the circular orbit displaced by feeding a magnetic field of the dynamic magnetic field feed device passes.

13. A particle therapy system comprising the particle beam accelerator according to any one of claims 1 to 12.

FIG. 1

# FIG. 2

## FIG. 3

EP 4 358 653 A1

*FIG. 4*

*FIG. 5*

## FIG. 6

## FIG. 7

# FIG. 8

## INTERNATIONAL SEARCH REPORT

| International application No. |
|---|
| **PCT/JP2022/002575** |

**A. CLASSIFICATION OF SUBJECT MATTER**

*H05H 7/04*(2006.01)i; *H05H 7/10*(2006.01)i; *H05H 13/00*(2006.01)i; *A61N 5/10*(2006.01)i
FI:   H05H7/10; H05H7/04; A61N5/10 H; H05H13/00

According to International Patent Classification (IPC) or to both national classification and IPC

**B. FIELDS SEARCHED**

Minimum documentation searched (classification system followed by classification symbols)

H05H7/04; H05H7/10; H05H13/00; A61N5/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2020-035728 A (HITACHI, LTD.) 05 March 2020 (2020-03-05)<br>   paragraphs [0065]-[0075], [0105]-[0107], [0120], fig. 4, 10 | 1-13 |
| Y | D. W. Storm et al. NEVIS SYNCHROCYCLOTRON BEAM STATUS REPORT. IEEE<br>Transactions on Nuclear Science, 01 June 1975, vol. NS-22, no. 3, pp. 1408-1410<br>   p. 1408, fig. 1 | 1-13 |
| Y | US 2014/0028220 A1 (MASSACHUSETTS INSTITUTE OF TECHNOLOGY) 30 January<br>2014 (2014-01-30)<br>   paragraphs [0092], [0093], fig. 12 | 3, 8, 11-12 |
| A | Novel Variable-energy Accelerator fot Particle Beam Therapy. Hitachi Review, March 2020,<br>vol. 69, no. 3, 386, 387, p. 133<br>   p. 133 | 1-13 |

☐ Further documents are listed in the continuation of Box C.      ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **25 March 2022** | **05 April 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

| International application No. |
| --- |
| **PCT/JP2022/002575** |

| Patent document cited in search report | | | Publication date (day/month/year) | Patent family member(s) | | | Publication date (day/month/year) |
| --- | --- | --- | --- | --- | --- | --- | --- |
| JP | 2020-035728 | A | 05 March 2020 | WO 2020/044604 A1 paragraphs [0065]-[0075], [0105]-[0107], [0120], fig. 4, 10 | | | |
| US | 2014/0028220 | A1 | 30 January 2014 | WO 2014/018706 A1 paragraphs [0104], [0105], [0114], fig. 12 | | | |
| | | | | EP | 2878180 | A | |
| | | | | CN | 104663003 | A | |
| | | | | HK | 1205621 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6714146 B